Europäisches Patentamt

European Patent Office

Office européen des brevets

(19)

(11) Veröffentlichungsnummer : **0 150 309**
**B1**

(12) **EUROPÄISCHE PATENTSCHRIFT**

(45) Veröffentlichungstag der Patentschrift :
28.06.89

(21) Anmeldenummer : 84114172.4

(22) Anmeldetag : 23.11.84

(51) Int. Cl.⁴ : **G 01 N 33/573,**
**G 01 N 33/577,**
**C 12 Q 1/40//**
**C12P21/00,**
**C12N15/00,**
**C12N5/00**

(54) **Hybridoma-Antikörper.**

(30) Priorität : 25.11.83 DE 3342736

(43) Veröffentlichungstag der Anmeldung :
07.08.85 Patentblatt 85/32

(45) Bekanntmachung des Hinweises auf die Patenterteilung : 28.06.89 Patentblatt 89/26

(84) Benannte Vertragsstaaten :
AT BE CH DE FR GB IT LI LU NL SE

(56) Entgegenhaltungen :
EP—A— 0 093 436
US—A— 4 012 285
PATENTS ABSTRACTS OF JAPAN, Band 8, Nr. 15 (C-206)[1452], 21. Januar 1984; & JP - A - 58 183 098 (WAKO JUNYAKU KOGYO K.K.) 26.10.1983
CLINICAL CHEMISTRY, Band 28, Nr. 11, November 1982, Seiten 2201-2205, Washington, US; E.-O. HÄGELE u.a.: "Mechanism of action of human pancreatic and salivary alpha-amylase on alpha-4-nitrophenyl maltoheptaoside substrate"
CLINICAL CHEMISTRY, Band 25, Nr. 3, März 1979, Seiten 481-483, Easton, Pennsylvania, US; K.J. WHITLOW u.a.: "Maltotetraose as a substrate for enzyme-coupled assay of amylase activity in serum and urine"
Die Akte enthält technische Angaben, die nach dem Eingang der Anmeldung eingereicht wurden und die nicht in dieser Patentschrift enthalten sind.

(73) Patentinhaber : BOEHRINGER MANNHEIM GMBH
Patentabteilung, Abt. E Sandhofer Strasse 112-132
Postfach 31 01 20
D-6800 Mannheim 31 Waldhof (DE)

(72) Erfinder : Naujoks, Kurt Walter, Dr. rer. nat.
Hiltlstrasse 15
D-8035 Gauting (DE)
Erfinder : Gerhardt, Willie, Dr. med.
Hallandsgatan 9
S-25252 Helsingborg (SE)
Erfinder : Hübner-Parajsz, Christa, Dr. rer. nat.
Marienstrasse 11
D-8132 Tutzing (DE)
Erfinder : Wulff, Karl, Dr. rer. nat.
Pütrichstrasse 18
D-8120 Weilheim (DE)
Erfinder : Jungfer, Herbert, Dr. med.
Beringerweg 10
D-8132 Tutzing (DE)
Erfinder : Lenz, Helmut, Dr. rer. nat.
Waldschmidtstrasse 7
D-8132 Tutzing (DE)
Erfinder : Albert, Winfried, Dr. phil.
Moosstrasse 10
D-8121 Pähl (DE)
Erfinder : Wahlefeld, August Wilhelm, Dr. rer. nat.
Alpenblickstrasse 25
D-8126 Hohenpeissenberg (DE)

(74) Vertreter : Huber, Bernhard, Dipl.-Chem. et al
Patentanwälte H. Weickmann, Dr. K. Fincke F.A.
Weickmann, B. Huber Dr. H. Liska, Dr. J. Prechtel
Möhlstrasse 22 Postfach 860 820
D-8000 München 86 (DE)

## Beschreibung

Die Erfindung betrifft ein Verfahren zur spezifischen Bestimmung von Pankreas-α-Amylase neben Speichel-α-Amylase.

α-Amylase (E.C.3.2.1.1) baut 1,4-d-glucosidisch verknüpfte Oligo- und Polysaccharid überwiegend durch zufällige Hydrolyse der 1,4-α-glycosidischen Bindungen zu Maltose und Malto-oligosacchariden ab. Das Enzym hat neben der industriellen Fermentationstechnik erhebliche Bedeutung im Rahmen der klinischen Analytik und Diagnostik. Bei zahlreichen Erkrankungen verändert sich nämlich der α-Amylasegehalt in den Körperflüssigkeiten wie Serum, Harn oder Duodenalsekret beträchtlich. Im Körper kommen jedoch im wesentlichen zwei α-Amylaseenzyme vor, das Pankreasenzym und das Speichelenzym. Da diagnostische Bedeutung nur dem Pankreasenzym zukommt, stellt sich die Aufgabe, diese beiden (neben selten und nur in geringer Menge auftretenden weiteren Isoenzymen) α-Amylasen analytisch zu differenzieren. Die Schwierigkeit besteht hierbei darin, daß die beiden multiplen Formen ähnlichen Aufbau besitzen und immunologisch identisch sind (K. Lorentz, Laboratoriumsblätter 32 : 118 (1982)). Zur Eliminierung der Aktivität des Speichelenzyms ist es bekannt, Adsorbtion an Anionenaustauscher, Hemmung durch ein Weizenprotein oder Elektrophorese bzw. Elektrofokusierung anzuwenden. Diese Verfahren sind jedoch entweder in ihrer Trennwirkung unbefriedigend oder für eine Routinediagnostik zu aufwendig. Unter den genannten Methoden ist allein das in Clin. Chem. 28/7, 1525-1527 (1982) beschriebene Verfahren der Hemmung des Enzyms vom Speicheltyp durch einen aus Weizenkeimen gewonnenen Inhibitor mit einem für die Routinediagnose akzeptablen Zeitaufwand verbunden, jedoch ist die Selektivität unbefriedigend. Auch bei der optimalen Inhibitorkonzentration bleiben etwa 13 % der Aktivität des Speicheltyp-Enzyms erhalten, während die Aktivität des Pankreasenzyms auf etwa 81 % verringert wird.

Der Erfindung liegt daher die Aufgabe zugrunde, diesen Nachteil zu beseitigen und ein Verfahren und ein Reagenz zu schaffen, welches eine rasche, einfache und zuverlässige Bestimmung der Pankreas-α-Amylase neben der α-Amylase vom Speicheltyp in Körperflüssigkeiten ermöglicht.

Gelöst wird diese Aufgabe erfindungsgemäß durch ein Verfahren zur spezifischen Bestimmung von Pankreas-α-Amylase neben Speichel-α-Amylase in Körperflüssigkeiten, insbesondere in Serum, Plasma, Duodenalsaft oder Urin, durch Umsetzung mit einem System zur Nachweis von α-Amylase in Gegenwart eines Hemmstoffes für Speichel-α-Amylase, welches dadurch gekennzeichnet ist, daß anstelle eines Hemmstoffs ein monoklonaler Antikörper gegen Speichel-α-Amylase mit einer Kreuzreaktivität von 5 % oder weniger gegenüber Pankreas-α-Amylase zugesetzt wird.

Das erfindungsgemäße Verfahren beruht auf der sehr überraschenden Auffindung eines monoklonalen Antikörpers mit sehr geringer Kreuzreaktivität gegenüber dem Pankreasenzym. Dies war nicht zu erwarten, da es bekannt war, daß das Speichel- und das Pankreasenzym immunologisch identisch sind (Gerhard Pfleiderer, Lab. Med 7, 189-193 ; K. Lorentz loc. cit.). So gibt Morton K. Schwarz in « Immunoassay of Enzymes — an Overview » (1983), Seite 4 bis 9, z. B. an, daß Antikörper gegen humane Speichel-α-Amylase das Enzym vom Speicheltyp zu 78 %, das Pankreasenzym zu 75 % hemmen. Es war daher nicht vorhersehbar, daß es möglich sein würde, ein Verfahren zu entwickeln, welches eine praktisch quantitative Unterscheidung der beiden Enzyme auf immunologischer Basis ermöglichen würde.

Der neue, für das Verfahren der Erfindung verwendete monoklonale Antikörper wurde bei NCACC unter den Nummern Klon 79 = NCACC 84111305, Klon 1A813E10 = NCACC 84111304, Klon 1A814A7 = NCACC 84111303, Klon 32C516E3 = NCACC 84111302 und Klon 32C518F11 = NCACC 84111301 hinterlegt (National Collection of Animal Cell Culture, Porton Down, GB). Er läßt sich erfindungsgemäß erhalten durch Immunisierung von Ratten oder Mäusen mit nativer oder modifizierter Speichel-α-Amylase unter Verwendung von Aluminiumhydroxid und Bordatella pertussis als Adjuvanz, wobei man bei Verwendung von nativer Speichel-α-Amylase die Immunisierung mindestens siebenmal über mindestens 9 Monate hinweg durchführt und bei Verwendung von modifizierter Speichel-α-Amylase mindestens zwei Immunisierungen in vivo, gefolgt von mindestens einer Immunisierung in vitro, bei der B-Lymphocyten in von Thymocyten konditioniertem Medium gezüchtet werden, durchführt, Fusion von B-Lymphocyten der immunisierten Tiere mit Myeloma-Zellen, Klonierung und Kultivierung der so gebildeten Hybridzellen und Isolierung der monoklonalen Antikörper aus letzteren erhalten wurde.

Verwendet man natives Enzym, so eignen sich hierzu die handelsüblichen, elektrophoretisch homogenen Präparate. Chemisch modifizierte Speichel-α-Amylase läßt sich ebenfalls nach bekannten Methoden der Enzymmodifikation erhalten, wie sie z. B. in der DE-B-25 43 994 näher beschrieben sind. Geeignete Modifizierungsmittel sind beispielsweise N-Bromsuccinimid (NBS) unter Oxidation von Tryptophangruppen am Protein (BBA 143 (1967) 462-472), Carboxymethylierung mit Jodacetat (IAA), die hauptsächlich am Histidin angreift bzw. Nitrierung mit Tetranitromethan (J. Biol. Chem. 238 (1963) 3307), Diazotierung mit diazotierter Sulfanilsäure (Meth. Enzymol. 25 (1972) 515-531) sowie Umsetzung mit Dinitrofluorbenzol (DNFB) (J. Biol. Chem. 243 (1968) 5344-253). Am besten geeignet erwies sich dabei, abgesehen vom nativen Enzym, das mit DNFB modifizierte Enzym.

Die Fusionierung erfolgt nach dem bekannten Verfahren von Koehler und Milstein (Nature 256 (1975) 495-997). Die hierbei gebildeten Hybridzellen werden in üblicher Weise kloniert, z. B. unter Verwendung eines handelsüblichen Zellsorters, und die erhaltenen Klone, welche den gewünschten monoklonalen Antikörper bilden, gezüchtet. Aufgrund des krebsartigen Wachsens der Hybridzellen lassen sich diese auf unbestimmte Zeit weiterkultivieren und produzieren den gewünschten monoklonalen Antikörper in beliebiger Menge. Mit den so erhaltenen monoklonalen Antikörpern kann die Speichel-α-Amylase aus Körperflüssigkeiten quantitativ absorbiert werden, so daß die verbleibende Amylaseaktivität der Pankreas-α-Amylase zuzuordnen ist.

Für das erfindungsgemäße Bestimmungsverfahren können die monoklonalen Antikörper als solche oder ihre entsprechende immunologische Eigenschaften aufweisenden Fragmente (Fc-Fragmente) verwendet werden. Unter dem Begriff « monoklonaler Antikörper » werden hier daher auch die Fragmente verstanden. Sowohl der komplette Antikörper als auch seine Fragmente können in immobilisierter Form verwendet werden.

Der erfindungsgemäß verwendete monoklonale Antikörper weist überraschenderweise gegenüber der Pankreas-α-Amylase eine Kreuzreaktivität von 5 % oder weniger auf und erreicht in vielen Fällen eine Kreuzreaktivität von nur noch 1 %. Er eignet sich daher anstelle des bisher bekannten, aus Weizenkeimen gewonnenen Hemmstoffes zur spezifischen Bestimmung der Pankreas-α-Amylase in Körperflüssigkeiten.

Die Bestimmung der α-Amylase als solche erfolgt nach den hierfür bekannten Methoden. Da der erfindungsgemäße monoklonale Antikörper selektiv die α-Amylase vom Speicheltyp bindet und sie so der Enzymaktivitätsbestimmung entzieht, entsprechen die bei der α-Amylase-Bestimmung in Gegenwart des monoklonalen Antikörpers erhaltenen Werte der dem Pankreasenzym alleine zukommenden Aktivität.

Bevorzugt wird das erfindungsgemäße Verfahren mit einem System zum Nachweis von α-Amylase durchgeführt, welches eine Maltopolyose mit 4 bis 7 Glucoseresten im Molekül, Maltosephosphorylase, β-Phosphoglucomutase, Glucose-6-phosphatdehydrogenase und NAD enthält.

Ein weiteres, im Rahmen der Erfindung bevorzugt verwendetes System zum Nachweis der α-Amylase besteht aus Nitrophenylmaltopolyose mit 4 bis 7 Glucoseresten im Molekül und α-Glucosidase.

Ein weiteres bevorzugtes Nachweissystem für α-Amylase besteht aus mit bestimmbaren Gruppen modifizierter Stärke. Der Begriff modifizierte Stärke umfaßt beispielsweise Stärke, die mit bestimmbaren Gruppen modifiziert ist, z. B. das als « Phadebas » handelsübliche Produkt der Firma Pharmazia, Schweden sowie das in der DE-OS 28 12 154 beschriebene Produkt sowie im Abbauverhalten veränderte Stärke, beispielsweise Carboxymethylstärke und Grenzdextrine. Alle diese Systeme sind bekannt und bedürfen daher hier keiner näheren Beschreibung.

Für die Durchführung des erfindungsgemäßen Verfahrens wird die Probeflüssigkeit mit dem erfindungsgemäßen Antikörper zweckmäßig in Form einer Suspension versetzt und dann das Unlösliche abgetrennt, vorzugsweise durch kurzes Zentrifugieren. Der klare Überstand wird dann im üblichen α-Amylasetest eingesetzt.

Im Rahmen des erfindungsgemäßen Reagenz kann der monoklonale Antikörper, sowohl in kompletter Form als auch in Form der Fragmente, auch auf einem festen Träger fixiert vorliegen, beispielsweise auf immunosorptivem Papier oder der Oberfläche von Kunststoffröhrchen bzw. -schläuchen. Auf diese Weise wird die α-Amylase vom Speicheltyp an den Träger, also die feste Phase, gebunden und in der flüssigen Phase kann daher ohne weitere Trennung die Bestimmung der Restaktivität, welche auf das Pankreasenzym zurückzuführen ist, durchgeführt werden.

Besonders gute Ergebnisse werden im Rahmen der Erfindung erhalten, wenn ein monoklonales Antikörperpräparat verwendet wird, welches aus den monoklonalen Antikörpern, die durch mehrere verschiedene Klone produziert werden, gemischt ist.

Da der aus dem monoklonalen Antikörper und seinem Antigen, der Speichel-α-Amylase, gebildete Komplex löslich ist, falls der monoklonale Antikörper nicht in immobilisierter Form verwendet wird, besteht eine weitere Möglichkeit zu seiner Abtrennung darin, zusätzlich ein präzipitierendes Agens für den monoklonalen Antikörper oder für die von den Versuchstieren bei der Immunisierung gebildeten Antikörpern zuzusetzen. Hierbei bildet sich ein unlöslicher Komplex, der Speichel-α-Amylase, monoklonalen Antikörper und präzipitierendes Agens enthält.

Als präzipitierendes Agens wird vorzugsweise ein Antikörper gegen den monoklonalen Antikörper oder für die von den Versuchstieren bei der Immunisierung gebildeten Antikörper (also ein Anti-Antikörper) verwendet. Eine andere Möglichkeit besteht in der Zugabe von Protein A, vorzugsweise in immobilisierter Form.

Eine zweckmäßige Methode, diese Ausführungsform des erfindungsgemäßen Verfahrens zu praktizieren, besteht darin, zuerst einen Komplex aus monoklonalem Antikörper und Anti-Antikörper zu bilden und diesen dann der zu untersuchenden Lösung zuzusetzen, welche die α-Amylasen enthält. Alternativ kann man jedoch zuerst nur den monoklonalen Antikörper zusetzen und, nach Inkubation zur Bildung des Antigen-Antikörper-Komplexes mit der Speichel-α-Amylase, dann den Anti-Antikörper zusetzen unter Bildung des unlöslichen Komplexes.

Für die Ausführungsform des erfindungsgemäßen Verfahrens unter Verwendung eines Anti-Antikörpers eignen sich prinzipiell alle gegen den monoklonalen Antikörper bzw. den von den Versuchstieren gebildeten Antikörper gerichteten Anti-Antikörper. Bevorzugt verwendet man bei Verwendung von Mäusen oder Ratten für die Erzeugung des Anti-Speichel-α-Amylase-Serums vom Schaf gebildete Anti-Antikörper.

3

Ein weiterer Gegenstand der Erfindung ist ein Reagenz zur spezifischen Bestimmung von Pankreas-α-Amylase neben Speichel-α-Amylase in Körperflüssigkeiten, insbesondere in Serum, Duodenalsaft, Plasma oder Urin, enthaltend ein System zum Nachweis von α-Amylase und einen Hemmstoff für Speichel-α-Amylase, welches dadurch gekennzeichnet ist, daß es anstelle eines Hemmstoffs einen monoklonalen Antikörper mit einer Kreuzreaktivität von 5 % oder weniger gegenüber Pankreas-α-Amylase enthält.

Hinsichtlich des im erfindungsgemäßen Reagenz enthaltenen Systems zum Nachweis von α-Amylase und der übrigen Bedingungen gelten die obigen Ausführungen bezüglich des Verfahrens entsprechend.

Die Erfindung ermöglicht eine einfache und rasche Bestimmung der Pankreas-α-Amylase neben α-Amylase vom Speicheltyp in Körperflüssigkeiten mit hoher Spezifität und verbessert damit die Möglichkeiten der klinischen Diagnostik.

Die folgenden Beispiele erläutern die Erfindung weiter.

## Beispiel 1 .

A) Balb/c-Mäuse werden mit 100 μg humaner Speichelamylase in Aluminiumhydroxid mit BORDETELLA PERTUSSIS immunisiert. In ca. achtwöchigem Rhytmus wird mit je 50 μg humaner Speichelamylase im gleichen Adjuvant drei- bis viermal weiterimmunisiert. Vier Tage vor Fusion wird die letzte Immunisierung mit 50 μg Speichelamylase in physiologischer Kochsalzlösung intravenös durchgeführt.

B) Die Fusion der Milzzellen mit Ag8.653 (ATCC CRL 1580) oder SP2/O (ATCC CRL 1581) Myelomzellen wird nach dem Standardverfahren gemäß J. of Imm. Meth. Vol. 39, 285-308 durchgeführt. Das Fusionsverhältnis Milz zu Myelomzellen ist 5 : 1. Die Fusionsprodukte werden auf 10 24er-Kulturschalen (von Costar) angesät und mit $5 \times 10^4$ peritoneal-exsudat-Zellen pro Kulturnapf gefüttert. Positive Primärkulturen (siehe Beispiel 3) werden 3 bis 4 Wochen nach Fusion mit Hilfe eines Fluoreszenz-aktivierten Zellsorters kloniert. Die Zellen werden einzeln in 96er Costar-Platten abgelegt und mit $2 \times 10^4$ peritoneal-exsudat-Zellen gefüttert.

## Beispiel 2

Modifizierung der α-Amylase mit Dinitrofluorbenzol

20 μmol Speichelamylase/l und 20 mmol Dinitrofluorbenzol/l (gelöst in wenig Ethanol) werden vermischt und bei Raumtemperatur für 10 Minuten inkubiert. Die Lösung wird dann 20 Stunden gegen Phosphatpuffer (50 mmol/l, pH 6,8) dialysiert (Kühlraum). Das Dialysat zeigt dann im Differenzspektrum eine Extinktionszunahme von 0,070 bei der Wellenlänge 360 nm. Die Lösung wird bis zur Immunisierung eingefroren. Die Proteinkonzentration im Dialysat beträgt 15 μmol/l.

Mit der so erhaltenen modifizierten α-Amylase wurden Mäuse, wie im Beispiel 1 beschrieben, immunisiert. Die mit modifizierter Amylase immunisierten Mäuse sterben alle nach .der zweiten Immunisierung. Deshalb werden die Milzzellen dieser Mäuse nach dem ersten Boost in Kultur genommen und nach der Methode von Luben (Molec. Immunology Vol. 17 (1980) 635-639) noch vier Tage « in vitro » in der Gegenwart von 100 μg Antigen in 30 ml Medium nachimmunisiert. Nach 4 Tagen werden die Zellen wie die nach Beispiel 1 erhaltenen Milzzellen fusioniert und weiterbehandelt.

## Beispiel 3

Um die Konzentration und Spezifität amylasebindender Antikörper im Serum immunisierter Mäuse oder im Kulturüberstand der Hybridzellen oder in Ascites zu erfassen, wird ein ELISA als Testprinzip verwendet. Dazu werden 96er ELISA-Platten (Fa. NUNC) mit Schaf-anti-Maus-Fc-Antikörper beschichtet. Zur Reduktion unspezifischer Bindung wird mit Rinderserum-Albumin (2 %ig in physiologischer Kochsalzlösung) nachbeschichtet. Danach wird mit der den Antikörper enthaltenden Probe oder mit verschiedenen Verdünnungen derselben inkubiert.

Die daran anschließende Inkubation wird entweder mit Speichelamylase-Peroxidase-(POD)-Konjugat oder mit Pankreasamylase-POD-Konjugat durchgeführt. Die Aktivität der gebundenen POD wird mit ABTS (2,2'-Azinodi-[3-ethylbenzthiazolinsulfonat (6)]) (pH 4,4) bestimmt und die Extinktion direkt als Maß für die gebundenen Maus-Antikörper genommen. Zur Bestimmung der Kreuzreaktion wird für jede Probe die Bindung von Speichel- und Pankreasamylase-POD getrennt bestimmt und die mit Speichelamylase-POD erhaltene Extinktion als 100 % Bindung gesetzt. Die gleichzeitig erhaltene Extinktion mit Pankreas-Amylase-POD ergibt in % der Extinktion der Speichelamylase-POD die Kreuzreaktion der Probe.

Es wurden 4 Klone gefunden, bei welchen überhaupt keine Bindung der Pankreas-α-Amylase-POD erfolgte und 5 Klone mit Kreuzreaktionen zwischen 1,5 und unter 5 %. Die nach dem Screening ausgesuchten Klone werden expandiert.

RPMI-Medium ist beschrieben in J.A.M.A. 199 (1957) 519 und ist handelsüblich.

Beispiel 4

Immobilisierung des monoklonalen Antikörpers (MAK) durch Schaf-anti-Maus-AK und nachfolgende Bindung der Speichelamylase

Ascites aus Maus
Schaf-anti-Maus-AK (IgGFcγ) : 19 g/l
Phosphatpuffer, pH = 7,0 ; 0,05 mol/l
Phosphatpuffer, pH = 7,0 ; 0,05 mol/l mit 2 % Rinderserumalbumin
humane Speichelamylase : 1 000 U/l (μMol Substratumsatz/ml/min (37 °C) mit 4-Nitrophenylmaltoheptaosid als Substrat)
humane Pankreasamylase : 1 000 U/l
Essigsäure : 0,5 mol/l
Dikaliumhydrogensulfatlösung : 2 mol/l

Der MAK aus Ascites wird im Verhältnis 1 : 100 mit Schaf-anti-Maus Antikörper gemischt. Diese Mischung wird 1 : 2 mit Phosphatpuffer verdünnt und 15 Minuten bei 37 °C geschüttelt. Das entstandene Präzipitat wird zweimal mit Puffer gewaschen und dann in Essigsäure (ungefähr 1/5 des Startvolumens) gelöst. Nach 5 Minuten Schütteln wird im Verhältnis 1 : 2 $K_2HPO_4$-Lösung zugegeben. Das Präzipitat entsteht wieder. Dieses wird zweimal mit Phosphatpuffer mit RSA (Rinderserumalbumin) und danach zweimal nur mit Puffer gewaschen. Anschließend wird das Präzipitat in 1/5 des Startvolumens mit Puffer suspendiert. 50 μl des Präzipitates werden zu 50 μl Speichel- bzw. Pankreas-Amylase gegeben und 15 Minuten bei Raumtemperatur inkubiert. Danach wird das Präzipitat abzentrifugiert und der Überstand gemäß Beispiel 7 weiterverarbeitet. Als Kontrolle dient eine Amylaseprobe, die anstatt mit Präzipitat mit 50 μl Puffer behandelt wurde.

Beispiel 5

Bindung des MAK an Speichelamylase und nachfolgende Präzipitation mit Schaf-anti-Maus Antikörper.

Der Ascites mit monoklonalen Antikörpern wird im Verhältnis 1 : 50 mit Puffer verdünnt. 50 μl dieser Lösung werden mit 50 μl Speichel- bzw. Pankreas-Amylase 15 Minuten bei Raumtemperatur geschüttelt. Als Kontrolle dient einerseits eine Mischung aus Ascitesverdünnung mit Puffer (Leerwert des Ascites) und andererseits eine Mischung aus Amylase und Puffer (Kontrolle der Amylaseaktivität). Anschließend werden 50 μl des präzipitierenden Anti-Antikörpers zugegeben. Nach 10 Minuten bei Raumtemperatur wird das Präzipitat abzentrifugiert und der Überstand gemäß Beispiel 7 weiterverarbeitet.

Beispiel 6

Immobilisierung des MAK durch Schaf-anti-Maus-Antikörper und nachfolgende Bindung von humaner Speichelamylase aus einer Mischung von Speichel- und Pankreasamylase.

Entsprechend Beispiel 5 wird eine Mischung aus humaner Pankreas- und Speichelamylase (je 1 000 U/l) mit vorbehandeltem Präzipitat inkubiert. Nach Zentrifugation wird der Überstand entsprechend Beispiel 7 weiterverarbeitet.

Beispiel 7

Bestimmung der verbleibenden Pankreasamylase.

50 μl Überstand gemäß Beispiel 4 bis 6 werden zu 1 ml handelsüblichem Reagenz zur Bestimmung von α-Amylase mit 4-Nitrophenylmaltoheptaosid als Substrat (Boehringer Mannheim, Kat. Best. Nr. 568589) gegeben. Die Aktivität des Überstandes wird nach Anleitung bei 37 °C bestimmt. Die Restaktivitäten ermitteln sich als Prozent Aktivität bezogen auf die jeweils mitlaufenden Kontrollen. Die Restaktivität bei Beispiel 4 beträgt mit Speichel-Amylase 0 bis 5 % und mit Pankreasamylase 70 bis 95 %. Die Versuche gemäß Beispiel 5 ergeben Restaktivitäten mit Speichelamylase von 0 bis 5 % und mit Pankreas-Amylase von 80 bis 95 %. Die gleichzeitige Anwesenheit beider Enzyme (Beispiel 6) ermöglicht die selektive Fällung der Speichelamylase, wobei die Pankreasamylase aktiv bleibt und zu 80 bis 95 % bestimmt werden kann.

**Patentansprüche** (für die Vertragsstaaten : BE, CH, DE, FR, GB, IT, LI, LU, NL, SE)

1. Verfahren zur spezifischen Bestimmung von Pankreas-α-Amylase neben Speichel-α-Amylase in Körperflüssigkeiten, insbesondere in Serum, Plasma, Duodenalsaft oder Urin, durch Umsetzung mit einem System zum Nachweis von α-Amylase in Gegenwart eines Hemmstoffes für Speichel-α-Amylase,

5

EP 0 150 309 B1

dadurch gekennzeichnet, daß anstelle eines Hemmstoffs ein monoklonaler Antikörper gegen Speichel-α-Amylase mit einer Kreuzreaktivität von 5 % oder weniger gegenüber Pankreas-α-Amylase zugesetzt wird.

2. Verfahren nach Anspruch 1, dadurch gekennzeichnet, daß ein monoklonaler Antikörper verwendet wird, der durch Immunisierung von Ratten oder Mäusen mit nativer oder modifizierter Speichel-α-Amylase unter Verwendung von Aluminiumhydroxid und Bordatella pertussis als Adjuvanz, wobei man bei Verwendung von nativer Speichel-α-Amylase die Immunisierung mindestens siebenmal über mindestens 9 Monate hinweg durchführt und bei Verwendung von modifizierter Speichel-α-Amylase mindestens zwei Immunisierungen in vivo, gefolgt von mindestens einer Immunisierung in vitro, bei der B-Lymphocyten in von Thymocyten konditioniertem Medium gezüchtet werden, durchführt, Fusion von B-Lymphocyten der immunisierten Tiere mit Myeloma-Zellen, Klonierung und Kultivierung der so gebildeten Hybridzellen und Isolierung der monoklonalen Antikörper aus letzteren erhalten wurde.

3. Verfahren nach einem der Ansprüche 1 oder 2, dadurch gekennzeichnet, daß man den monoklonalen Antikörper in immobilisierter Form einsetzt.

4. Verfahren nach einem der Ansprüche 1 bis 3, dadurch gekennzeichnet, daß man zusätzlich ein präzipitierendes Agens zugibt und den gebildeten unlöslichen Komplex aus Speichel-α-Amylase, monoklonalem Antikörper und Anti-Antikörper von der Lösung abtrennt.

5. Verfahren nach Anspruch 4, dadurch gekennzeichnet, daß man als präzipitierendes Agens einen Antikörper (Anti-Antikörper) für den monoklonalen Antikörper oder für die von den Versuchstieren gebildeten Antikörpern zugibt.

6. Verfahren nach Anspruch 4, dadurch gekennzeichnet, daß man als präzipitierendes Agens Protein A zugibt.

7. Verfahren nach Anspruch 4, 5 oder 6, dadurch gekennzeichnet, daß man einen vorgebildeten Komplex aus monoklonalem Antikörper und Anti-Antikörper zusetzt.

8. Verfahren nach Anspruch 4, dadurch gekennzeichnet, daß man zuerst den monoklonalen Antikörper zusetzt, inkubiert und dann den Komplex durch Zusatz des Anti-Antikörpers unlöslich macht.

9. Verfahren nach einem der Ansprüche 4 bis 8, dadurch gekennzeichnet, daß man Anti-Antikörper vom Schaf verwendet.

10. Verfahren nach einem der vorhergehenden Ansprüche, dadurch gekennzeichnet, daß als System zum Nachweis von α-Amylase eine Maltopolyose mit 4 bis 7 Glucoseresten, Maltosephosphorylase, β-Phosphoglucomutase, Glucose-6-phosphatdehydrogenase und NAD verwendet wird.

11. Verfahren nach einem der Ansprüche 1 bis 9, dadurch gekennzeichnet, daß als System zum Nachweis von α-Amylase Nitrophenylmaltopolyose mit 4 bis 7 Glucoseeinheiten im Molekül zusammen mit α-Glucosidase verwendet wird.

12. Verfahren nach einem der Ansprüche 1 bis 9, dadurch gekennzeichnet, daß als System zum Nachweis von α-Amylase Stärke, die mit bestimmbaren Gruppen modifiziert ist, verwendet wird.

13. Reagenz zur spezifischen Bestimmung von Pankreas-α-Amylase neben Speichel-α-Amylase in Körperflüssigkeiten, insbesondere in Serum, Duodenalsaft, Plasma oder Urin, enthaltend ein System zum Nachweis von α-Amylase und einen Hemmstoff für Speichel-α-Amylase, dadurch gekennzeichnet, daß es anstelle eines Hemmstoffs einen monoklonalen Antikörper gegen Speichel-α-Amylase mit einer Kreuzreaktivität von 5 % oder weniger gegenüber Pankreas-α-Amylase enthält.

14. Reagenz nach Anspruch 13, dadurch gekennzeichnet, daß es als System zum Nachweis von α-Amylase eine Maltopolyose mit 4 bis 7 Glucoseresten, Maltosephosphorylase, β-Phosphoglucomutase, Glucose-6-phosphatdehydrogenase und NAD enthält.

15. Reagenz nach Anspruch 13, dadurch gekennzeichnet, daß es als System zum Nachweis von α-Amylase eine Nitrophenylmaltopolyose mit 4 bis 7 Glucoseeinheiten und α-Glucosidase enthält.

16. Reagenz nach Anspruch 13, dadurch gekennzeichnet, daß es als System zum Nachweis von α-Amylase eine mit bestimmbaren Gruppen modifizierte Stärke enthält.

17. Monoklonaler Antikörper gegen Speichel-α-Amylase, gekennzeichnet durch eine Kreuzreaktion von 5 % oder weniger gegenüber Pankreas-α-Amylase, Subklasse IgG 2 b Kappa, erhältlich durch Immunisierung von Ratten oder Mäusen mit nativer oder modifizierter Speichel-α-Amylase unter Verwendung von Aluminiumhydroxid und Bordatella pertussis als Adjuvanz, wobei man bei Verwendung von nativer Speichel-α-Amylase die Immunisierung mindestens siebenmal über mindestens 9 Monate hinweg durchführt und bei Verwendung von modifizierter Speichel-α-Amylase mindestens zwei Immunisierungen in vivo, gefolgt von mindestens einer Immunisierung in vitro, bei der B-Lymphocyten in von Thymocyten konditioniertem Medium gezüchtet werden, durchführt, Fusion von B-Lymphocyten der immunisierten Tiere mit Myeloma-Zellen, Klonierung und Kultivierung der so gebildeten Hybridzellen und Isolierung der monoklonalen Antikörper aus letzteren.

**Patentansprüche** (für den Vertragsstaat AT)

1. Verfahren zur spezifischen Bestimmung von Pankreas-α-Amylase neben Speichel-α-Amylase in Körperflüssigkeiten, insbesondere in Serum, Plasma, Duodenalsaft oder Urin, durch Umsetzung mit einem System zum Nachweis von α-Amylase in Gegenwart eines Hemmstoffes für Speichel-α-Amylase, dadurch gekennzeichnet, daß anstelle eines Hemmstoffs ein monoklonaler Antikörper gegen Speichel-α-Amylase mit einer Kreuzreaktivität von 5 % oder weniger gegenüber Pankreas-α-Amylase zugesetzt wird.

6

EP 0 150 309 B1

2. Verfahren nach Anspruch 1, dadurch gekennzeichnet, daß ein monoklonaler Antikörper verwendet wird, der durch Immunisierung von Ratten oder Mäusen mit nativer oder modifizierter Speichel-α-Amylase unter Verwendung von Aluminiumhydroxid und Bordatella pertussis als Adjuvanz, wobei man bei Verwendung von nativer Speichel-α-Amylase die Immunisierung mindestens siebenmal über mindestens 9 Monate hinweg durchführt und bei Verwendung von modifizierter Speichel-α-Amylase mindestens zwei Immunisierungen in vivo, gefolgt von mindestens einer Immunisierung in vitro, bei der B-Lymphocyten in von Thymocyten konditioniertem Medium gezüchtet werden, durchführt, Fusion von B-Lymphocyten der immunisierten Tiere mit Myeloma-Zellen, Klonierung und Kultivierung der so gebildeten Hybridzellen und Isolierung der monoklonalen Antikörper aus letzteren erhalten wurde.

3. Verfahren nach einem der Ansprüche 1 oder 2, dadurch gekennzeichnet, daß man den monoklonalen Antikörper in immobilisierter Form einsetzt.

4. Verfahren nach einem der Ansprüche 1 bis 3, dadurch gekennzeichnet, daß man zusätzlich ein präzipitierendes Agens zugibt und den gebildeten unlöslichen Komplex aus Speichel-α-Amylase, monoklonalem Antikörper und Anti-Antikörper von der Lösung abtrennt.

5. Verfahren nach Anspruch 4, dadurch gekennzeichnet, daß man als präzipitierendes Agens einen Antikörper (Anti-Antikörper) für den monoklonalen Antikörper oder für die von den Versuchstieren gebildeten Antikörpern zugibt.

6. Verfahren nach Anspruch 4, dadurch gekennzeichnet, daß man als präzipitierendes Agens Protein A zugibt.

7. Verfahren nach Anspruch 4, 5 oder 6, dadurch gekennzeichnet, daß man einen vorgebildeten Komplex aus monoklonalem Antikörper und Anti-Antikörper zusetzt.

8. Verfahren nach Anspruch 4, dadurch gekennzeichnet, daß man zuerst den monoklonalen Antikörper zusetzt, inkubiert und dann den Komplex durch Zusatz des Anti-Antikörpers unlöslich macht.

9. Verfahren nach einem der Ansprüche 4 bis 8, dadurch gekennzeichnet, daß man Anti-Antikörper vom Schaf verwendet.

10. Verfahren nach einem der vorhergehenden Ansprüche, dadurch gekennzeichnet, daß als System zum Nachweis von α-Amylase eine Maltopolyose mit 4 bis 7 Glucoseresten, Maltosephosphorylase, β-Phosphoglucomutase, Glucose-6-phosphatdehydrogenase und NAD verwendet wird.

11. Verfahren nach einem der Ansprüche 1 bis 9, dadurch gekennzeichnet, daß als System zum Nachweis von α-Amylase Nitrophenylmaltopolyose mit 4 bis 7 Glucoseeinheiten im Molekül zusammen mit α-Glucosidase verwendet wird.

12. Verfahren nach einem der Ansprüche 1 bis 9, dadurch gekennzeichnet, daß als System zum Nachweis von α-Amylase Stärke, die mit bestimmbaren Gruppen modifiziert ist, verwendet wird.

13. Reagenz zur spezifischen Bestimmung von Pankreas-α-Amylase neben Speichel-α-Amylase in Körperflüssigkeiten, insbesondere in Serum, Duodenalsaft, Plasma oder Urin, enthaltend ein System zum Nachweis von α-Amylase und einen Hemmstoff für Speichel-α-Amylase, dadurch gekennzeichnet, daß es anstelle eines Hemmstoffs einen monoklonalen Antikörper gegen Speichel-α-Amylase mit einer Kreuzreaktivität von 5 % oder weniger gegenüber Pankreas-α-Amylase enthält.

14. Reagenz nach Anspruch 13, dadurch gekennzeichnet, daß es als System zum Nachweis von α-Amylase eine Maltopolyose mit 4 bis 7 Glucoseresten, Maltosephosphorylase, β-Phosphoglucomutase, Glucose-6-phosphatdehydrogenase und NAD enthält.

15. Reagenz nach Anspruch 13, dadurch gekennzeichnet, daß es als System zum Nachweis von α-Amylase eine Nitrophenylmaltopolyose mit 4 bis 7 Glucoseeinheiten und α-Glucosidase enthält.

16. Reagenz nach Anspruch 13, dadurch gekennzeichnet, daß es als System zum Nachweis von α-Amylase eine mit bestimmbaren Gruppen modifizierte Stärke enthält.


**Claims** (for the Contracting States : BE, CH, DE, FR, GB, IT, LI, LU, NL, SE)

1. Process for the specific determination of pancreas α-amylase in body fluids in the presence of saliva α-amylase, especially in serum, plasma, duodenal juice or urine, by reaction with a system for the detection of α-amylase in the presence of an inhibiting agent for saliva α-amylase, characterised in that, instead of an inhibiting agent, there is added a monoclonal antibody against saliva α-amylase with a cross-reactivity of 5 % or less towards pancreas α-amylase.

2. Process according to claim 1, characterised in that a monoclonal antibody is used which has been obtained by immunisation of rats or mice with native or modified saliva α-amylase with the use of aluminium hydroxide or Bordatella pertussis as adjuvant, whereby, in the case of use of native saliva α-amylase, one carries out the immunisation at least seven times over at least 9 months and, in the case of the use of modified saliva α-amylase, carries out at least two immunisations in vivo, followed by at least one immunisation in vitro in which the B-lymphocytes are cultured in medium conditioned by thymocytes, fusion of B-lymphocytes of the immunised animals with myeloma cells, cloning and culturing of the so-formed hybrid cells and isolation of the monoclonal antibodies from the latter.

3. Process according to one of claims 1 or 2, characterised in that one uses the monoclonal antibodies in immobilised form.

4. Process according to one of claims 1 to 3, characterised in that one additionally adds a

7

precipitating agent and separates the insoluble complex formed from saliva α-amylase, monoclonal antibody and anti-antibody from the solution.

5. Process according to claim 4, characterised in that, as precipitating agent, one adds an antibody (anti-antibody) for the monoclonal antibodies or for the antibodies formed by the experimental animals.

6. Process according to claim 4, characterised in that one adds protein A as precipitating agent.

7. Process according to claim 4, 5 or 6, characterised in that one adds a pre-formed complex of monoclonal antibodies and anti-antibodies.

8. Process according to claim 4, characterised in that one first adds the monoclonal antibodies, incubates and then makes the complex insoluble by addition of the anti-antibody.

9. Process according to one of claims 4 to 8, characterised in that one uses anti-antibodies from sheep.

10. Process according to one of the preceding claims, characterised in that, as system for the detection of α-amylase, there is used a maltopolyose with 4 to 7 glucose residues, maltose phosphorylase, β-phosphoglucomutase, glucose-6-phosphate dehydrogenase and NAD.

11. Process according to one of claims 1 to 9, characterised in that, as system for the detection of α-amylase, there is used nitrophenylmaltopolyose with 4 to 7 glucose units in the molecule, together with α-glucosidase.

12. Process according to one of claims 1 to 9, characterised in that, as system for the detection of α-amylase, starch which is modified with determinable groups is used.

13. Reagent for the specific determination of pancreas α-amylase in the presence of saliva α-amylase in body fluids, especially in serum, duodenal juice, plasma or urine, containing a system for the detection of α-amylase and an inhibiting agent for saliva α-amylase, characterised in that, instead of an inhibiting agent, it contains a monoclonal antibody against saliva α-amylase with a cross-reactivity of 5 % or less towards pancreas α-amylase.

14. Reagent according to claim 13, characterised in that, as system for the detection of α-amylase, it contains a maltopolyose with 4 to 7 glucose residues, maltose phosphorylase, β-phosphoglucomutase, glucose-6-phosphate dehydrogenase and NAD.

15. Reagent according to claim 13, characterised in that, as system for the detection of α-amylase, it contains a nitrophenylmaltopolyose with 4 to 7 glucose units and α-glucosidase.

16. Reagent according to claim 13, characterised in that, as system for the detection of α-amylase, it contains a starch modified with determinable groups.

17. Monoclonal antibody against saliva α-amylase, characterised by a cross-reaction of 5 % or less towards pancreas α-amylase, subclass IgG 2 b kappa, obtainable by immunisation of rats or mice with native or modified saliva α-amylase with use of aluminium hydroxide or Bordatella pertussis as adjuvant, whereby, in the case of the use of native saliva α-amylase, one carries out the immunisation at least seven times over at least 9 months and, in the case of the use of modified saliva α-amylase, carries out at least two immunisations in vivo, followed by at least one immunisation in vitro, in which B-lymphocytes are cultured in medium conditioned by thymocytes, fusion of B-lymphocytes of the immunised animals with myeloma cells, cloning and culturing of the so-formed hybrid cells and isolation of the monoclonal antibodies from the latter.


**Claims** (for the Contracting State AT)

1. Process for the specific determination of pancreas α-amylase in body fluids in the presence of saliva α-amylase, especially in serum, plasma, duodenal juice or urine, by reaction with a system for the detection of α-amylase in the presence of an inhibiting agent for saliva α-amylase, characterised in that, instead of an inhibiting agent, there is added a monoclonal antibody against saliva α-amylase with a cross-reactivity of 5 % or less towards pancreas α-amylase.

2. Process according to claim 1, characterised in that a monoclonal antibody is used which has been obtained by immunisation of rats or mice with native or modified saliva α-amylase with the use of aluminium hydroxide or Bordatella pertussis as adjuvant, whereby, in the case of use of native saliva α-amylase, one carries out the immunisation at least seven times over at least 9 months and, in the case of the use of modified saliva α-amylase, carries out at least two immunisations in vivo, followed by at least one immunisation in vitro in which the B-lymphocytes are cultured in medium conditioned by thymocytes, fusion of B-lymphocytes of the immunised animals with myeloma cells, cloning and culturing of the so-formed hybrid cells and isolation of the monoclonal antibodies from the latter.

3. Process according to one of claims 1 or 2, characterised in that one uses the monoclonal antibodies in immobilised form.

4. Process according to one of claims 1 to 3, characterised in that one additionally adds a precipitating agent and separates the insoluble complex formed from saliva α-amylase, monoclonal antibody and anti-antibody from the solution.

5. Process according to claim 4, characterised in that, as precipitating agent, one adds an antibody (anti-antibody) for the monoclonal antibodies or for the antibodies formed by the experimental animals.

6. Process according to claim 4, characterised in that one adds protein A as precipitating agent.

7. Process according to claim 4, 5 or 6, characterised in that one adds a pre-formed complex of monoclonal antibodies and anti-antibodies.

8. Process according to claim 4, characterised in that one first adds monoclonal antibodies, incubates and then makes the complex insoluble by addition of the anti-antibody.

9. Process according to one of claims 4 to 8, characterised in that one uses anti-antibodies from sheep.

10. Process according to one of the preceding claims, characterised in that, as system for the detection of α-amylase, there is used a maltopolyose with 4 to 7 glucose residues, maltose phosphorylase, β-phosphoglucomutase, glucose-6-phosphate dehydrogenase and NAD.

11. Process according to one of claims 1 to 9, characterised in that, as system for the detection of α-amylase, there is used nitrophenylmaltopolyose with 4 to 7 glucose units in the molecule, together with α-glucosidase.

12. Process according to one of claims 1 to 9, characterised in that, as system for the detection of α-amylase, starch which is modified with determinable groups is used.

13. Reagent for the specific determination of pancreas α-amylase in the presence of saliva α-amylase in body fluids, especially in serum, duodenal juice, plasma or urine, containing a system for the detection of α-amylase and an inhibiting agent for saliva α-amylase, characterised in that, instead of an inhibiting agent, it contains a monoclonal antibody against saliva α-amylase with a cross-reactivity of 5 % or less towards pancreas α-amylase.

14. Reagent according to claim 13, characterised in that, as system for the detection of α-amylase, it contains a maltopolyose with 4 to 7 glucose residues, maltose phosphorylase, β-phosphoglucomutase, glucose-6-phosphate dehydrogenase and NAD.

15. Reagent according to claim 13, characterised in that, as system for the detection of α-amylase, it contains a nitrophenylmaltopolyose with 4 to 7 glucose units and α-glucosidase.

16. Reagent according to claim 13, characterised in that, as system for the detection of α-amylase, it contains a starch modified with determinable groups.

**Revendications** (pour les Etats contractants : BE, CH, DE, FR, GB, IT, LI, LU, NL, SE)

1. Procédé pour la détermination spécifique de l'α-amylase du pancréas à côté de l'α-amylase de la salive dans des liquides biologiques, en particulier dans le sérum, le plasma, le suc du duodénal ou l'urine, par réaction avec un système pour la mise en évidence de l'α-amylase en présence d'une substance inhibitrice pour l'α-amylase de la salive, caractérisé en ce que, au lieu d'une substance inhibitrice, on ajoute un anticorps monoclonal contre l'α-amylase de la salive ayant une réactivité croisée de 5 % ou moins vis-à-vis de l'α-amylase du pancréas.

2. Procédé selon la revendication 1, caractérisé en ce qu'on utilise un anticorps monoclonal qui a été obtenu par immunisation de rats ou de souris avec de l'α-amylase de salive native ou modifiée en utilisant de l'hydroxyde d'aluminium et du Bordetella pertussis en tant qu'adjuvant, dans le cas de l'utilisation d'α-amylase de salive native, l'immunisation étant effectuée au moins sept fois en l'espace d'au moins 9 mois et dans le cas de l'utilisation d'α-amylase de salive modifiée, au moins deux immunisations in vivo, suivies d'au moins une immunisation in vitro dans laquelle des lymphocytes B sont cultivés dans du milieu conditionné par des thymocytes étant effectuées, fusion de lymphocytes B des animaux immunisés avec des cellules myélomateuses, clonage et culture des cellules hybrides ainsi formées et isolement des anticorps monoclonaux à partir de ces dernières.

3. Procédé selon l'une des revendications 1 ou 2, caractérisé en ce qu'on met en œuvre l'anticorps monoclonal sous forme immobilisée.

4. Procédé selon l'une des revendications 1 à 3, caractérisé en ce qu'on ajoute de plus un agent précipitant et en ce que l'on sépare de la solution le complexe d'α-amylase de salive, d'anticorps monoclonal et d'anti-anticorps formé.

5. Procédé selon la revendication 4, caractérisé en ce qu'on ajoute, en tant qu'agent précipitant, un anticorps (anti-anticorps) pour l'anticorps monoclonal ou pour les anticorps formés par les animaux d'essai.

6. Procédé selon la revendication 4, caractérisé en ce que l'on ajoute, en tant qu'agent précipitant, de la protéine A.

7. Procédé selon la revendication 4, 5 ou 6, caractérisé en ce qu'on ajoute un complexe préformé d'anticorps monoclonal et d'anti-anticorps.

8. Procédé selon la revendication 4, caractérisé en ce qu'on ajoute d'abord l'anticorps monoclonal, incube puis rend le complexe insoluble par addition de l'anti-anticorps.

9. Procédé selon l'une des revendications 4 à 8, caractérisé en ce que l'on utilise de l'anti-anticorps de mouton.

10. Procédé selon l'une des revendications précédentes, caractérisé en ce que l'on utilise comme système pour la mise en évidence de l'α-amylase un maltopolyose avec 4 à 7 restes de glucose, de la maltose-phosphorylase, de la β-phosphoglucomutase, de la glucose-6-phosphatedéshydrogénase et du NAD.

11. Procédé selon l'une des revendications 1 à 9, caractérisé en ce que l'on utilise, comme système pour la mise en évidence de l'α-amylase, du nitrophénylmaltopolyose avec 4 à 7 unités glucose dans la molécule, ensemble avec de l'α-glucosidase.

12. Procédé selon l'une des revendications 1 à 9, caractérisé en ce que l'on utilise comme système pour la mise en évidence de l'α-amylase, de l'amidon qui est modifié par des groupes déterminables.

13. Réactif pour la détermination spécifique de l'α-amylase du pancréas à côté d'α-amylase de la salive dans des liquides biologiques, en particulier dans le sérum, le suc du duodénal, le plasma ou l'urine, contenant un système pour la mise en évidence de l'α-amylase et une substance inhibitrice pour l'α-amylase de la salive, caractérisé en ce qu'il contient, à la place d'une substance inhibitrice, un anticorps monoclonal contre l'α-amylase de la salive, avec une réactivité croisée de 5 % ou moins vis-à-vis de l'α-amylase du pancréas.

14. Réactif selon la revendication 13, caractérisé en ce qu'il contient, en tant que système pour la mise en évidence de l'α-amylase, un maltopolyose avec 4 à 7 restes de glucose, de la maltosephosphorylase, de la glucose-6-phosphatedéshydrogénase et du NAD.

15. Réactif selon la revendication 13, caractérisé en ce qu'il contient, en tant que système pour la mise en évidence de l'α-amylase, un nitrophénylmaltopolyose avec 4 à 7 unités glucose et de l'α-glucosidase.

16. Réactif selon la revendication 13, caractérisé en ce qu'il contient, en tant que système pour la mise en évidence de l'α-amylase, un amidon modifié par des groupes déterminables.

17. Anticorps monoclonal contre l'α-amylase de la salive, caractérisé par une réaction croisée de 5 % ou moins vis-à-vis de l'α-amylase du pancréas, sous-classe IgG 2 b Kappa, qui peut être obtenu par immunisation de rats ou de souris par de l'α-amylase de salive native ou modifiée en utilisant de l'hydroxyde d'aluminium et du Bordetella pertussis comme adjuvant, dans le cas de l'utilisation d'α-amylase de salive native, l'immunisation étant effectuée au moins sept fois en l'espace d'au moins 9 mois et dans le cas de l'utilisation d'α-amylase de salive modifiée, au moins deux immunisations in vivo, suivies d'au moins une immunisation in vitro dans laquelle des lymphocytes B sont cultivés dans du milieu conditionné par des thymocytes, étant effectuées, fusion de lymphocytes B des animaux immunisés avec des cellules myélomateuses, clonage et culture des cellules hybrides ainsi formées et isolement des anticorps monoclonaux à partir de ces dernières.

**Revendications** (pour l'Etat contractant AT)

1. Procédé pour la détermination spécifique de l'α-amylase du pancréas à côté de l'α-amylase de la salive dans des liquides biologiques, en particulier dans le sérum, le plasma, le suc du duodénal ou l'urine, par réaction avec un système pour la mise en évidence de l'α-amylase en présence d'une substance inhibitrice pour l'α-amylase de la salive, caractérisé en ce que, au lieu d'une substance inhibitrice, on ajoute un anticorps monoclonal contre l'α-amylase de la salive ayant une réactivité croisée de 5 % ou moins vis-à-vis de l'α-amylase du pancréas.

2. Procédé selon la revendication 1, caractérisé en ce qu'on utilise un anticorps monoclonal qui a été obtenu par immunisation de rats ou de souris avec de l'α-amylase de salive native ou modifiée en utilisant de l'hydroxyde d'aluminium et du Bordetella pertussis en tant qu'adjuvant, dans le cas de l'utilisation d'α-amylase de salive native, l'immunisation étant effectuée au moins sept fois en l'espace d'au moins 9 mois et dans le cas de l'utilisation d'α-amylase de salive modifiée, au moins deux immunisations in vivo, suivies d'au moins une immunisation in vitro dans laquelle des lymphocytes B sont cultivés dans du milieu conditionné par des thymocytes étant effectuées, fusion de lymphocytes B des animaux immunisés avec des cellules myélomateuses, clonage et culture des cellules hybrides ainsi formées et isolement des anticorps monoclonaux à partir de ces dernières.

3. Procédé selon l'une des revendications 1 ou 2, caractérisé en ce qu'on met en œuvre l'anticorps monoclonal sous forme immobilisée.

4. Procédé selon l'une des revendications 1 à 3, caractérisé en ce qu'on ajoute de plus un agent précipitant et en ce que l'on sépare de la solution le complexe d'α-amylase de salive, d'anticorps monoclonal et d'anti-anticorps formé.

5. Procédé selon la revendication 4, caractérisé en ce qu'on ajoute, en tant qu'agent précipitant, un anticorps (anti-anticorps) pour l'anticorps monoclonal ou pour les anticorps formés par les animaux d'essai.

6. Procédé selon la revendication 4, caractérisé en ce que l'on ajoute, en tant qu'agent précipitant, de la protéine A.

7. Procédé selon la revendication 4, 5 ou 6, caractérisé en ce qu'on ajoute un complexe préformé d'anticorps monoclonal et d'anti-anticorps.

8. Procédé selon la revendication 4, caractérisé en ce qu'on ajoute d'abord l'anticorps monoclonal, incube puis rend le complexe insoluble par addition de l'anti-anticorps.

9. Procédé selon l'une des revendications 4 à 8, caractérisé en ce que l'on utilise de l'anti-anticorps de mouton.

10. Procédé selon l'une des revendications précédentes, caractérisé en ce que l'on utilise comme système pour la mise en évidence de l'α-amylase un maltopolyose avec 4 à 7 restes de glucose, de la maltose-phosphorylase, de la β-phosphoglucomutase, de la glucose-6-phosphatedéshydrogénase et du NAD.

11. Procédé selon l'une des revendications 1 à 9, caractérisé en ce que l'on utilise, comme système pour la mise en évidence de l'α-amylase, du nitrophénylmaltopolyose avec 4 à 7 unités glucose dans la molécule, ensemble avec de l'α-glucosidase.

12. Procédé selon l'une des revendications 1 à 9, caractérisé en ce que l'on utilise comme système pour la mise en évidence de l'α-amylase, de l'amidon qui est modifié par des groupes déterminables.

13. Réactif pour la détermination spécifique de l'α-amylase du pancréas à côté d'α-amylase de la salive dans des liquides biologiques, en particulier dans le sérum, le suc du duodénal, le plasma ou l'urine, contenant un système pour la mise en évidence de l'α-amylase et une substance inhibitrice pour l'α-amylase de la salive, caractérisé en ce qu'il contient, au lieu d'une substance inhibitrice, un anticorps monoclonal contre l'α-amylase de la salive, avec une réactivité croisée de 5 % ou moins vis-à-vis de l'α-amylase du pancréas.

14. Réactif selon la revendication 13, caractérisé en ce qu'il contient, en tant que système pour la mise en évidence de l'α-amylase, un maltopolyose avec 4 à 7 restes de glucose, de la maltosephosphorylase, de la glucose-6-phosphatedéshydrogénase et du NAD.

15. Réactif selon la revendication 13, caractérisé en ce qu'il contient, en tant que système pour la mise en évidence de l'α-amylase, un nitrophénylmaltopolyose avec 4 à 7 unités glucose et de l'α-glucosidase.

16. Réactif selon la revendication 13, caractérisé en ce qu'il contient, en tant que système pour la mise en évidence de l'α-amylase, un amidon modifié par des groupes déterminables.